# FASCICULE DE BREVET EUROPEEN

(11) **EP 2 385 893 B1**
(45) Date de publication et mention de la délivrance du brevet: **04.05.2016**
(21) Numéro de dépôt: 09764532.9
(22) Date de dépôt: 07.12.2009
(51) Int. Cl.: B29C 45/28

(54) **SYSTÈME D'INJECTION DE MATIERE THERMOPLASTIQUE**
EINSPRITZVORRICHTUNG FUER THERMOPLASTE
INJECTION DEVICE FOR THERMOPLASTIC MATERIALS

(30) Priorité: 09.12.2008 FR 0858394
(43) Date de publication de la demande: 16.11.2011
(73) Titulaire: RUNIPSYS EUROPE, 73420 Mery (FR)
(72) Inventeur: DERICHE, Eric, F-73420 Mery (FR)
(74) Mandataire: Regimbeau
(86) Numéro de dépôt international: PCT/EP2009/066508
(87) Numéro de publication internationale: WO 2010/066671

(56) Documents cités:
- FR-A- 2 821 010
- US-A- 4 712 995

## Description

### DOMAINE DE L'INVENTION

La présente invention concerne un système d'injection de matière thermoplastique dans une empreinte de moulage, et un procédé de fabrication d'un tel système.

### ARRIERE PLAN DE L'INVENTION

Un système d'injection de type « bloc chaud » ou « à canaux chauds » comporte habituellement :
- un distributeur délimitant un canal de distribution de matière thermoplastique et comportant une sortie de matière thermoplastique,
- une buse d'injection définissant au moins une portion d'un passage de transit dont l'entrée est en liaison fluidique avec la sortie du canal de distribution, et dont la sortie débouche sensiblement dans l'empreinte de moulage,
- un obturateur monté à coulissement longitudinal à l'intérieur du passage de transit et occupant de manière alternée une position d'obturation et une position d'ouverture de celui-ci,
- des moyens de commande pour faire coulisser alternativement l'obturateur entre la position d'obturation et la position d'ouverture.

Les moyens de commande comprennent typiquement un vérin relié à l'obturateur.

Ce type de système d'injection comporte en outre des moyens d'alimentation aptes à pourvoir le distributeur en matière à injecter.

Pour injecter de manière satisfaisante la matière dans l'empreinte, la matière doit être maintenue à l'état fluide, cet état étant obtenu lorsque la matière est portée à une température limite déterminée, supérieure à la température de l'air ambiant.

A cet effet, le distributeur comporte de manière connue des moyens permettant de maintenir sa température, et par conséquent celle de la matière transitant dans son canal de distribution, à une température supérieure à la température limite de passage à l'état fluide de la matière.

La matière à l'état fluide est introduite dans le canal de distribution du distributeur par les moyens d'alimentation et pénètre dans le passage de transit de la buse d'injection.

Lorsque les moyens de commande amènent l'obturateur dans la position d'obturation, la sortie de la buse est obturée et la matière à injecter est retenue dans le passage de transit.

Lorsque les moyens de commande amènent l'obturateur dans la position d'ouverture, la sortie de la buse est ouverte et la matière est injectée dans l'empreinte.

De manière classique, ces moyens de commande sont situés à l'air ambiant sur la face du distributeur longitudinalement opposée à l'empreinte de moulage (ou « face arrière » du distributeur).

Or, le distributeur étant maintenu à une température élevée, il est indispensable de prévoir un refroidissement du vérin.

Ce refroidissement est typiquement obtenu au moyen d'une circulation d'eau au niveau du vérin.

Or, la mise en place de ce circuit d'eau est problématique car elle complique le système d'injection et elle est relativement onéreuse.

Par ailleurs, l'épaisseur du vérin contribue à augmenter sensiblement l'épaisseur du système d'injection.

Un des buts de l'invention est donc de concevoir un système d'injection dans lequel on puisse s'affranchir du refroidissement des moyens de commande de l'obturateur.

Un autre but de l'invention est de minimiser l'épaisseur totale du système d'injection. Un système d'injection tel que décrit dans le préambule de la revendication 1 est connu du brevet US 4712995.

### BREVE DESCRIPTION DE L'INVENTION

Conformément à l'invention, il est proposé un système d'injection, dans une empreinte de moulage, d'une matière thermoplastique à l'état fluide, comprenant :
- un distributeur propre à être maintenu à une température d'injection supérieure à la température limite au-delà de laquelle la matière se présente à l'état fluide, ledit distributeur comprenant un canal de distribution et au moins une sortie de matière thermoplastique,
- une buse d'injection définissant au moins une portion d'un passage de transit dont l'entrée est en liaison fluidique avec la sortie du canal de distribution, et dont la sortie débouche sensiblement dans l'empreinte de moulage,
- un obturateur monté à l'intérieur du passage de transit pour coulisser entre une position d'obturation de celui-ci et une position d'ouverture de celui-ci,
- des moyens de commande pour faire coulisser alternativement l'obturateur, ledit système étant caractérisé en ce que lesdits moyens de commande comprennent un vérin dont la tige est parallèle à la direction de coulissement de l'obturateur, fixé de manière déportée sur le distributeur par l'intermédiaire d'une poutre aplatie, et un levier agencé en basculement autour d'un axe de manière à transmettre à l'obturateur les mouvements de la tige du vérin.

De manière particulièrement avantageuse, ladite poutre porte l'axe de basculement du levier.

Selon un premier mode de réalisation de l'invention, la poutre est une pièce massive, c'est-à-dire monobloc. Elle présente ainsi une grande rigidité qui lui permet de respecter la tenue en fatigue imposée à un système pour l'injection d'une grande série de pièces (c'est-à-dire typiquement plus de 200.000 pièces).

Selon un deuxième mode de réalisation de l'invention, la poutre est formée d'un assemblage de pièces en tôle d'acier. Cette conception offre un bon compromis coût/rigidité pour une utilisation peu intensive de type petite série.

Selon d'autres caractéristiques de ce mode de réalisation, prises séparément ou en combinaison :
- ladite poutre comprend deux bras parallèles, une bride de vérin fixée sur le vérin, et une bride d'obturateur fixée sur le distributeur ;
- les bras et les brides d'obturateur et de vérin présentent des découpes complémentaires permettant leur emboîtement ;
- les bras et la bride d'obturateur présentent des découpes permettant leur centrage sur le pied de buse ;
- le levier comprend deux bras en tôle d'acier ;
- l'épaisseur des pièces en tôle est comprise entre 2 et 10 mm, de préférence entre 3 et 5 mm.

L'invention concerne également un dispositif de commande de coulissement d'un obturateur d'une buse d'injection, notamment pour un système d'injection tel que décrit précédemment, ledit dispositif étant caractérisé en ce qu'il comprend :
- une poutre aplatie pour fixer le vérin en position déportée sur le distributeur, et
- un levier agencé en basculement autour d'un axe de manière à transmettre à l'obturateur les mouvements de la tige du vérin.

Un autre objet de l'invention concerne un procédé de fabrication d'un système selon le deuxième mode de réalisation décrit plus haut, comprenant les étapes suivantes :
(a) découpe et, le cas échéant, pliage des pièces formant la poutre,
(b) assemblage desdites pièces et du levier,
(c) fixation de l'ensemble sur le vérin et le distributeur.

De manière avantageuse, l'étape (a) comprend également la découpe des bras du levier.

De préférence, l'étape (a) comprend des découpes par laser.

Enfin, l'étape (b) est avantageusement réalisée par emboîtement des pièces.

### BREVE DESCRIPTION DES DESSINS

D'autres caractéristiques et avantages de l'invention ressortiront de la description détaillée qui va suivre, en référence aux dessins annexés sur lesquels :
- la figure 1 est une vue éclatée de moyens de commande de l'obturateur conformes à un premier mode de réalisation de l'invention particulièrement adapté à une utilisation intensive de type grande série,
- la figure 2 est une vue éclatée des moyens de commande conformes à un deuxième mode de réalisation de l'invention destiné préférentiellement à une utilisation peu intensive de type prototype ou petite série,
- la figure 3 illustre les moyens de commande de la figure 2 en position assemblée,
- les figures 4 et 5 illustrent respectivement un détail d'assemblage de la tête d'obturateur de la figure 2, en vue de dessous et en vue de dessus,
- la figure 6 illustre les formes respectives de la poutre et de la bride permettant un emboîtement avec coopération de formes,
- la figure 7 illustre la bride de la figure 6 en position assemblée.

### DESCRIPTION DETAILLEE DE L'INVENTION

Un système d'injection selon l'invention comprend un vérin de commande de l'obturateur qui n'est pas fixé directement sur le distributeur, mais de manière déportée par rapport à celui-ci, par l'intermédiaire d'une poutre.

Cette implantation présente un double avantage.

Le premier est de diminuer l'épaisseur totale du système de l'ordre d'un tiers environ. En effet, le vérin est ainsi logé dans une cavité et seule l'épaisseur de la poutre reliant le vérin au distributeur contribue à l'épaisseur du système.

En outre, le vérin n'étant plus situé en contact du distributeur chaud, on peut s'affranchir du refroidissement par eau qui est nécessaire dans l'agencement de l'art antérieur.

Par ailleurs, les matériaux et épaisseurs de la poutre sont adaptés pour permettre une dissipation de la chaleur.

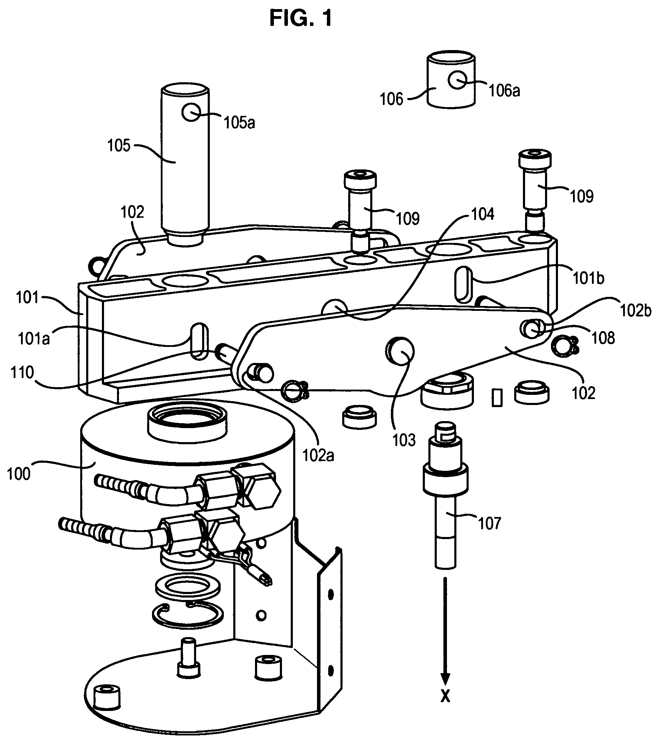

Selon un mode de réalisation de base de l'invention, les moyens de commande de l'obturateur comprennent, en référence à la figure 1 :
- un vérin 100, fixé de manière déportée sur le distributeur par l'intermédiaire d'une poutre 101 de forme aplatie,
- deux bras de levier 102 sensiblement symétriques, disposés de part et d'autre de la poutre 101, et montés en basculement sur celle-ci au moyen d'un axe 103 inséré dans un alésage traversant 104 situé sensiblement à mi-longueur de la poutre 101,
- une tête de vérin 105 montée sur le vérin 100 et mobile en translation dans une direction parallèle à la direction X de déplacement de l'obturateur,
- une tête d'obturateur 106 rigidement liée à l'obturateur 107.

La fixation du vérin 100 sur la poutre 101 est assurée par tout moyen connu, tel que des vis (non représentées sur la figure 1).

Les mouvements de la tête de vérin 105 sont transmis à la tête d'obturateur par l'intermédiaire des bras de levier 102.

En effet, la tête de vérin 105 et la tête d'obturateur 106 sont percées chacune d'un alésage respectivement 105a, 106a, à travers lequel passe un axe respectivement 110, 108.

Les axes 110 et 108 traversent respectivement la poutre 101 au niveau de deux trous oblongs 101 a, 101 b, et chacun des bras de levier 102 au niveau de deux trous oblongs 102a, 102b.

A son extrémité opposée au vérin 100, la poutre 101 est fixée sur le distributeur par deux vis 109.

Ainsi, lorsque le vérin impose une translation de la tête de vérin 105 vers le haut (dans la configuration de la figure 1), les leviers 102 basculent autour de l'axe 103 et entraînent la tête d'obturateur 106 en translation vers le bas, et occasionnent l'obturation de la buse.

Inversement, une translation de la tête de vérin 105 vers le bas entraîne la tête d'obturateur 106 en translation vers le haut, et permet ainsi l'ouverture de la buse.

La poutre 101 présente une forme aplatie, c'est-à-dire que sa longueur est supérieure à sa hauteur et que sa hauteur est inférieure ou égale à la hauteur du vérin.

A titre d'exemple non limitatif, la poutre 101 illustrée à la figure 1 présente une hauteur de l'ordre de 40 mm, une longueur d'environ 200 mm et une largeur d'environ 40 mm. Elle est associée par exemple à un vérin hydraulique présentant une hauteur de 40 mm environ.

Pour obtenir une rigidité suffisante et une robustesse compatible avec la durée de vie du système d'injection, la poutre 101 peut être réalisée par usinage d'un bloc d'acier. On précise que l'acier peut éventuellement subir un traitement à coeur ou en surface en vue d'améliorer ses propriétés.

Selon un autre mode de réalisation, la poutre 101 peut être réalisée en fonderie de fonte ou d'acier, le matériau pouvant en outre subir tout traitement approprié.

Ces modes de réalisation dans lesquels la poutre est massive, c'est-à-dire formée en une seule pièce, sont particulièrement adaptés lorsque le système d'injection est prévu pour la fabrication de grandes séries (c'est-à-dire devant assurer plus de 200.000 cycles d'injection). En effet, une telle poutre est apte à respecter le cahier des charges de tenue en fatigue pour le nombre de cycles prévu.

Par ailleurs, dans une recherche constante de diminution des coûts, notamment dans le cas de la fabrication de petites quantités de pièces (c'est-à-dire allant typiquement de 1000 pièces (prototypes) jusqu'à 200.000 pièces (petite série)), pour laquelle le coût du moule a un impact important sur le coût des pièces, une nouvelle conception de la poutre, visant à diminuer le coût de la poutre, a été définie.

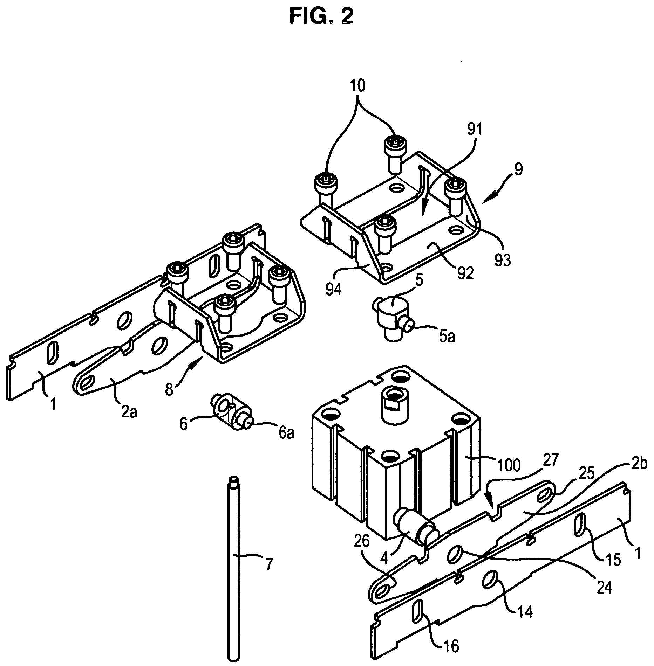

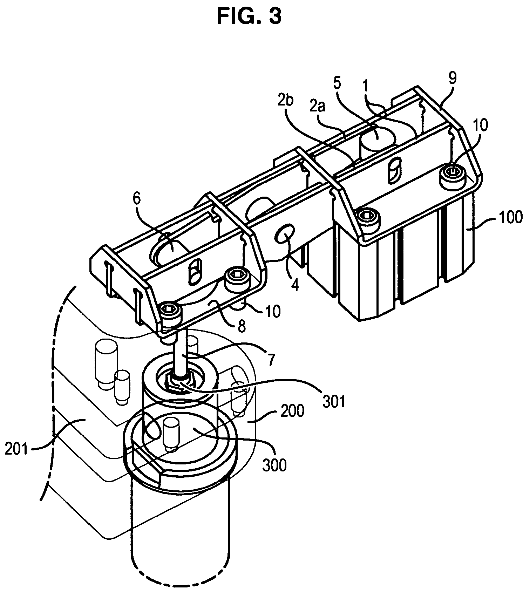

Selon une variante et en référence aux figures 2 et 3, la poutre est réalisée sous forme d'un assemblage de pièces de structure 1, 8 et 9 en tôle formées par découpe et pliage.

Deux bras 1 parallèles, sensiblement symétriques par rapport à un plan perpendiculaire à l'axe de basculement 4, ont pour fonction de relier rigidement une bride d'obturateur 8 et une bride de vérin 9.

Les bras 1 présentant une forme allongée sensiblement rectangulaire, on définit pour chaque bras son extrémité distale, qui est l'extrémité située du côté du vérin, et son extrémité proximale qui est l'extrémité située du côté du distributeur.

La bride d'obturateur 8 est fixée sur le distributeur 200 au moyen de quatre vis 10.

La bride de vérin 9 est fixée sur une face dite supérieure du vérin 100, perpendiculaire à la tige du vérin, par quatre vis 10.

En position assemblée, les bras 1 sont perpendiculaires à la face arrière du distributeur, sur laquelle est fixée la bride d'obturateur 8, et à la face supérieure du vérin.

Les bras 1 sont reliés par un axe de basculement 4.

Un levier comprenant deux bras 2a et 2b, parallèles et sensiblement symétriques par rapport à un plan perpendiculaire à l'axe de basculement 4, a pour fonction de transmettre le déplacement de la tige du vérin 100 à l'obturateur 7.

On définit pour chaque bras 2a, 2b du levier une extrémité distale, qui est l'extrémité située du côté du vérin, et une extrémité proximale qui est l'extrémité située du côté de l'obturateur.

Chaque bras 2a, 2b présente, vers son extrémité proximale, un alésage oblong 26 pour le passage de l'axe 6a de la tête d'obturateur 6.

L'alésage oblong 26 présente sa plus grande dimension perpendiculairement à la direction de coulissement de l'obturateur 7.

De même, chaque bras 2a, 2b présente vers son extrémité distale un alésage oblong 25 pour le passage de l'axe 5a de la tête de vérin 5, la plus grande dimension de l'alésage 25 étant dans une direction perpendiculaire à la direction X de coulissement de l'obturateur.

Par ailleurs, chaque bras 1 présente des alésages oblongs 16 et 15 respectivement pour le passage des axes 6a et 5a.

La plus grande dimension des alésages oblongs 15 et 16 est parallèle à la direction X de coulissement de l'obturateur 7.

Ainsi, la plus grande dimension des alésages 15 et 16 détermine la course de coulissement de l'obturateur 7.

La bride d'obturateur 8 présente une forme générale de U, avec une face 82 sensiblement carrée en contact avec la face arrière du distributeur 200, et deux branches parallèles 83 et 84 perpendiculaires à la face 82.

La face 82 est percée à proximité de ses coins de quatre alésages pour le passage des vis 10 de fixation sur le distributeur 200. Elle présente également une découpe centrale pour le passage de la tête d'obturateur 6.

En position assemblée, les branches 83 et 84 de la bride d'obturateur sont perpendiculaires aux bras 1.

La bride de vérin 9 présente également une forme générale de U, avec une face 92 sensiblement carrée en contact avec le vérin 100, et deux branches parallèles 93 et 94 perpendiculaires à la face 92.

La face 92 est percée à proximité de ses coins de quatre alésages pour le passage des vis 10 de fixation sur le vérin 100, et présente une découpe centrale 91 pour le passage de la tête de vérin 5.

Sur la figure 3, on peut observer par ailleurs le distributeur 200, un canal de distribution 201 débouchant dans le pied de buse 300, et le guide d'obturateur 301.

Les pièces de structure 1, 8 et 9 présentent des découpes avantageusement définies pour permettre un assemblage simple et un centrage des moyens de commande par rapport au pied de buse 300, afin d'éviter tout effort radial de l'obturateur 7 lorsqu'il coulisse dans le guide d'obturateur 301.

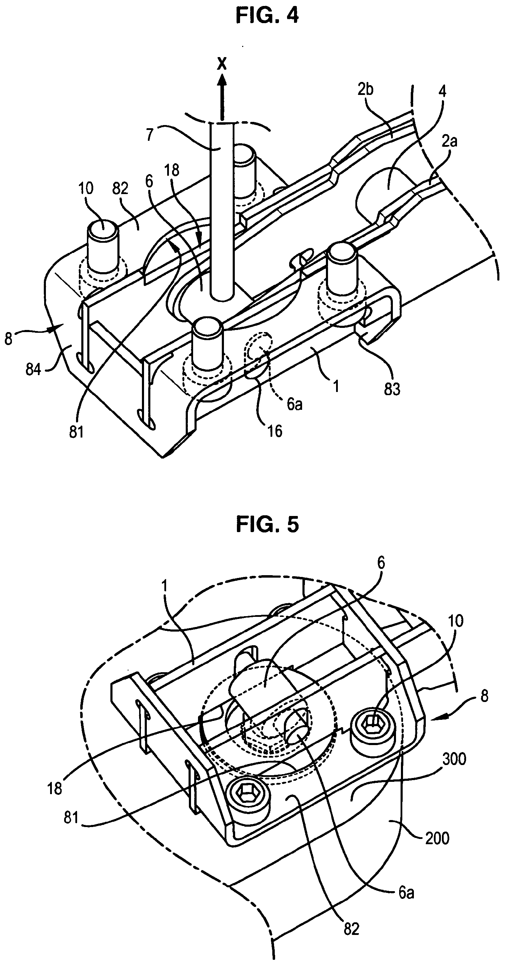

Ainsi, en référence à la figure 4, chaque bras 1 présente, sur le bord de son extrémité proximale orienté vers le distributeur, une découpe 18 en forme d'encoche rectangulaire.

Par ailleurs, la bride d'obturateur 8 présente, sur sa face 82 en contact avec le distributeur 200, une découpe circulaire 81 d'un diamètre légèrement supérieur à celui du pied de buse 300.

Ainsi, comme on peut le voir sur la figure 5, la bride 8 d'obturateur 8 et le bras 1 viennent coiffer le pied de buse 300, ce qui permet d'assurer un bon centrage de la tête d'obturateur 6 et de l'obturateur 7 par rapport au guide d'obturateur 301.

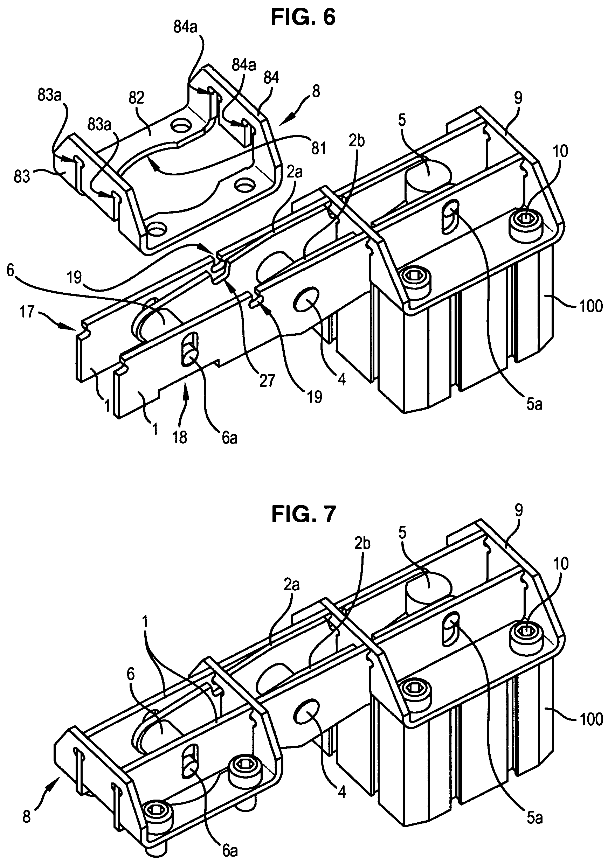

Par ailleurs, on peut observer sur la figure 6 que la bride 8 et le bras 1 présentent des découpes qui permettent l'assemblage de l'une sur l'autre par simple emboîtement.

Ainsi, la bride 8 présente, sur chacune de ses branches 83 et 84, deux découpes 83a, respectivement 84a.

Sur la branche 83, qui est celle située du côté de l'extrémité proximale des bras 1, la largeur des découpes 83a est légèrement supérieure à l'épaisseur de chaque bras 1.

Sur la branche 84, qui est celle située du côté de l'axe de basculement 4, la largeur des découpes 84a est légèrement supérieure à l'épaisseur du bras 1, du bras 2a ou 2b du levier, et du jeu entre ceux-ci.

Par ailleurs, chaque bras 1 présente, sur son bord opposé à la face du distributeur, deux encoches 17 et 19 destinées à recevoir la bride 8.

Les bras 2a et 2b du levier présentent quant à eux une encoche 27 permettant le montage de la bride 8 sans interférence avec le basculement du levier.

Ainsi, il suffit, pour assembler la bride 8, d'emboîter les découpes 83a et 84a de celle-ci sur les encoches 17, 19 des bras 1 et 27 des bras 2a et 2b, comme on peut le voir sur la figure 7.

La bride 9, les bras 1, 2a et 2b présentent des découpes similaires à celles qui viennent d'être décrites pour permettre l'emboîtement de la bride 9 sur les bras 1.

De préférence, les bras 1 et les bras 2a et 2b sont plans.

Ils peuvent donc être obtenu par une simple découpe (par exemple par laser) d'une tôle en prenant en compte les découpes telles que décrites plus haut, ainsi qu'un perçage des alésages 16, 14, 15 et 26, 24 et 25 respectivement prévus pour permettre le passage des axes 6a, 4 et 5a.

Les brides 8 et 9, qui présentent une forme générale de U, sont obtenues par une découpe à plat d'une tôle intégrant la réalisation des découpes qui viennent d'être décrites pour l'emboîtement sur les bras 1, ainsi que le perçage des alésages pour le passage des vis 10.

Après découpe, ces pièces sont pliées pour leur conférer la forme de U souhaitée.

De manière particulièrement avantageuse, toutes les pièces (bras 1, 2a, 2b et brides 8 et 9) sont découpées dans la même tôle, ce qui rend le procédé de fabrication encore plus simple et économique.

Pour garantir une rigidité suffisante des pièces de structure, il convient de choisir une tôle d'acier d'épaisseur comprise entre 2 et 10 mm, de préférence entre 3 et 5 mm.

La poutre ainsi formée et illustrée à la figure 3 présente une largeur d'environ 70 mm, une longueur de l'ordre de 180 mm et une hauteur de 30 mm.

Le vérin est par exemple un vérin pneumatique de 60 mm de haut par exemple.

Bien sûr, ces dimensions ne sont données qu'à titre d'exemple non limitatif.

On souligne que l'emploi de pièces en tôle permet mieux dissiper la chaleur susceptible d'être transmise au vérin.

L'ordre d'assemblage des moyens de commande de l'obturateur est le suivant :
(a) assemblage de la tête d'obturateur 6 sur l'obturateur 7, celui-ci étant au préalable inséré dans le guide d'obturateur 301 ;
(b) assemblage de la tête de vérin 5 sur la tige du vérin 100 ;
(c) emboîtement d'un premier bras 2a et d'un premier bras 1 sur les axes 6a et 5a de la tête d'obturateur 6 et de la tête de vérin 5 ;
(d) mise en place de l'axe de basculement 4 dans les alésages 24 et 14 du premier bras 2a et du premier bras 1 ;
(e) mise en place du deuxième bras 2b et du deuxième bras 1 sur les axes 6a, 4 et 5a ;
(f) emboîtement de la bride de vérin 9 sur les bras 1 et vissage des quatre vis 10 sur le vérin 1;
(g) emboîtement de la bride d'obturateur 8 sur les bras 1 et vissage des quatre vis 10 sur le distributeur 200.

On comprendra toutefois que cet ordre n'est donné qu'à titre d'exemple et qu'une séquence différente des étapes est envisageable.

La fabrication et l'assemblage de ce nouveau dispositif sont donc particulièrement simples et peu onéreux.

Ce nouveau dispositif permet ainsi de diminuer significativement le prix de revient des moyens de commande.

Enfin, il va de soi que les exemples que l'on vient de donner ne sont que des illustrations particulières en aucun cas limitatives quant aux domaines d'application de l'invention.

Notamment, d'autres formes de la poutre massive, des bras du levier, des pièces de structure et des découpes permettant de les emboîter peuvent être envisagées sans pour autant sortir du champ de la présente invention.

De même, d'autres modes d'assemblage du vérin sur la poutre, de la poutre sur le distributeur, et des pièces constituant la poutre lorsque celle-ci n'est pas massive, peuvent être employés.

Par ailleurs, le système illustré ne comprend qu'une buse d'injection, mais on comprend que l'invention s'applique également à un système comprenant une pluralité de buses d'injection, chacune équipée d'un obturateur et des moyens de commande dudit obturateur.

## Revendications

1. Système d'injection, dans une empreinte de moulage, d'une matière thermoplastique à l'état fluide, comprenant :
- un distributeur (200) propre à être maintenu à une température d'injection supérieure à la température limite au-delà de laquelle la matière se présente à l'état fluide, ledit distributeur comprenant un canal de distribution et au moins une sortie de matière thermoplastique,
- une buse d'injection définissant au moins une portion d'un passage de transit dont l'entrée est en liaison fluidique avec la sortie du canal de distribution, et dont la sortie débouche sensiblement dans l'empreinte de moulage,
- un obturateur (7) monté à l'intérieur du passage de transit pour coulisser entre une position d'obturation de celui-ci et une position d'ouverture de celui-ci,
- des moyens de commande pour faire coulisser alternativement l'obturateur (7), lesdits moyens de commande comprennent un vérin (100) dont la tige est parallèle à la direction (X) de coulissement de l'obturateur (7), un levier agencé en basculement autour d'un axe (4) de manière à transmettre à l'obturateur (7) les mouvements de la tige du vérin, **caractérise en ce que** le vérin est fixé de manière déportée sur le distributeur (200) par l'intermédiaire d'une poutre aplatie.

2. Système selon la revendication 1, **caractérisé en ce que** la poutre porte l'axe (4) de basculement du levier.

3. Système selon l'une des revendications 1 ou 2, **caractérisé en ce que** la poutre est une pièce massive (101).

4. Système selon l'une des revendications 1 ou 2, **caractérisé en ce que** la poutre est formée d'un assemblage de pièces (1, 8, 9) en tôle d'acier.

5. Système selon la revendication 4, **caractérisé en ce que** ladite poutre comprend deux bras (1) parallèles, une bride de vérin (9) fixée sur le vérin (100), et une bride d'obturateur (8) fixée sur le distributeur (200).

6. Système selon la revendication 5, **caractérisé en ce que** les bras (1) et les brides (8, 9) d'obturateur et de vérin présentent des découpes complémentaires (17, 19, 83a, 84a) permettant leur emboîtement.

7. Système selon l'une des revendications 5 ou 6, **caractérisé en ce que** les bras (1) et la bride d'obturateur (8) présentent des découpes (18, 81) permettant leur centrage sur le pied de buse (300).

8. Système selon l'une des revendications 4 à 7, **caractérisé en ce que** le levier comprend deux bras (2a, 2b) en tôle d'acier.

9. Système selon l'une des revendications 4 à 8, **caractérisé en ce que** l'épaisseur des pièces (1, 2, 8, 9) en tôle est comprise entre 2 et 10 mm, de préférence entre 3 et 5 mm.

10. Dispositif de commande de coulissement d'un obturateur d'une buse d'injection notamment pour un système d'injection selon l'une des revendications 1 à 9, **caractérisé en ce qu'**il comprend :
- une poutre aplatie pour fixer le vérin (100) en position déportée sur le distributeur (200), et
- un levier agencé en basculement autour d'un axe (4) de manière à transmettre à l'obturateur (7) les mouvements de la tige du vérin.

11. Procédé de fabrication du système selon l'une des revendications 4 à 9, **caractérisé en ce qu'**il comprend les étapes suivantes :
(a) découpe et, le cas échéant, pliage des pièces (1, 8, 9) formant la poutre,
(b) assemblage desdites pièces et du levier,
(c) fixation de l'ensemble sur le vérin (100) et le distributeur (200).

12. Procédé selon la revendication 11, **caractérisé en ce que** l'étape (a) comprend également la découpe des bras (2a, 2b) du levier.

13. Procédé selon l'une des revendications 11 ou 12, **caractérisé en ce que** l'étape (a) comprend des découpes par laser.

14. Procédé selon l'une des revendications 11 à 13, **caractérisé en ce que** l'étape (b) est réalisée par emboîtement des pièces.

## Patentansprüche

1. Einspritzsystem eines thermoplastischen Materials in fluidem Zustand in eine Abdruckform, umfassend:
- einen Verteiler (200), der imstande ist, auf einer Einspritztemperatur gehalten zu werden, die höher ist als die Grenztemperatur, jenseits der das Material in fluidem Zustand ist, wobei der Verteiler einen Verteilungskanal und mindestens einen Ausgang des thermoplastischen Materials umfasst,
- eine Einspritzdüse, die mindestens einen Abschnitt eines Transitdurchgangs definiert, dessen Eingang in fluidischer Verbindung mit dem Ausgang des Verteilungskanals ist und dessen Ausgang etwa in die Abdruckform ausmündet,
- einen Verschluss (7), der im Innern des Transitdurchgangs montiert ist, um zwischen einer Verschlussstellung desselben und einer geöffneten Stellung desselben zu gleiten,
- Steuermittel, damit der Verschluss (7) alternativ gleitet, wobei die Steuermittel einen Zylinder (100) umfassen, dessen Stange parallel zur Gleitrichtung (X) des Verschlusses (7) ist, wobei ein Hebel schwenkend um eine Achse (4) angeordnet ist, um an den Verschluss (7) die Bewegungen der Stange des Zylinders zu übertragen,
**dadurch gekennzeichnet, dass** der Zylinder mittels eines abgeflachten Balkens versetzt auf dem Verteiler (200) befestigt ist.

2. System nach Anspruch 1, **dadurch gekennzeichnet, dass** der Balken die Schwenkachse (4) des Hebels trägt.

3. System nach einem der Ansprüche 1 oder 2, **dadurch gekennzeichnet, dass** der Balken ein massives Teil (101) ist.

4. System nach einem der Ansprüche 1 oder 2, **dadurch gekennzeichnet, dass** der Balken von einer Montage von Teilen (1, 8, 9) aus Stahlblech gebildet ist.

5. System nach Anspruch 4, **dadurch gekennzeichnet, dass** der Balken zwei parallele Arme (1), einen auf dem Zylinder (100) befestigten Zylinderflansch (9) und einen auf dem Verteiler (200) befestigten Verschlussflansch (8) umfasst.

6. System nach Anspruch 5, **dadurch gekennzeichnet, dass** die Arme (1) und die Verschluss- und Zylinderflansche (8, 9) komplementäre Ausschnitte (17, 19, 83a, 84a) aufweisen, die ihr Einpassen erlauben.

7. System nach einem der Ansprüche 5 oder 6, **dadurch gekennzeichnet, dass** die Arme (1) und der Verschlussflansch (8) Ausschnitte (18, 81) aufweisen, die ihre Zentrierung auf dem Düsenfuß (300) erlauben.

8. System nach einem der Ansprüche 4 bis 7, **dadurch gekennzeichnet, dass** der Hebel zwei Arme (2a, 2b) aus Stahlblech umfasst.

9. System nach einem der Ansprüche 4 bis 8, **dadurch gekennzeichnet, dass** die Dicke der Blechteile (1, 2, 8, 9) zwischen 2 und 10 mm, vorzugsweise zwischen 3 und 5 mm, inklusive beträgt.

10. Steuervorrichtung des Gleitens eines Verschlusses einer Einspritzdüse insbesondere für ein Einspritzsystem nach einem der Ansprüche 1 bis 9, **dadurch gekennzeichnet, dass** sie umfasst:
- einen abgeflachten Balken, um den Zylinder (100) in versetzter Stellung auf dem Verteiler (200) zu befestigen, und
- einen Hebel, der um eine Achse (4) schwenkend angeordnet ist, um an den Verschluss (7) die Bewegungen der Stange des Zylinders zu übertragen.

11. Herstellungsverfahren des Systems nach einem der Ansprüche 4 bis 9, **dadurch gekennzeichnet, dass** es die folgenden Schritte umfasst:
(a) Ausschneiden und gegebenenfalls Falten der Teile (1, 8, 9), die den Balken bilden,
(b) Montage der Teile und des Hebels,
(c) Befestigen der Einheit auf dem Zylinder (100) und dem Verteiler (200).

12. Verfahren nach Anspruch 11, **dadurch gekennzeichnet, dass** der Schritt (a) ebenfalls das Ausschneiden der Arme (2a, 2b) des Hebels umfasst.

13. Verfahren nach einem der Ansprüche 11 oder 12, **dadurch gekennzeichnet, dass** der Schritt (a) Laserausschnitte umfasst.

14. Verfahren nach einem der Ansprüche 11 bis 13, **dadurch gekennzeichnet, dass** der Schritt (b) durch Einpassen der Teile durchgeführt wird.

## Claims

1. A system for injecting a thermoplastic material in the fluid state into a molding cavity, including:
- a manifold (200) capable of being held at an injection temperature greater than the final temperature beyond which the material occurs in the fluid state, said manifold including a distribution channel and at least one thermoplastic material outlet,
- an injection nozzle defining at least one portion of a transit passage the entrance whereof is fluidically connected with the outlet of the distribution channel, and the outlet whereof opens substantially into the molding cavity,
- a nozzle gate (7) mounted within the transit passage so as to slide between a position closing same and a position opening same,
- control means for causing the nozzle gate (7) to slide alternately,
said control means include a power cylinder (100) the rod whereof is parallel to the sliding direction (X) of the nozzle gate (7), a lever arranged to tilt about a trunnion (4) so as to transmit to the nozzle gate (7) the movements of the cylinder rod,
**characterized in that** the cylinder is secured in an offset fashion to the manifold (200) through a flattened beam.

2. A system according to claim 1, **characterized in that** the beam holds the tilting trunnion (4) of the lever.

3. A system according to either claim 1 or 2, **characterized in that** the beam is a solid part (101).

4. A system according to either claim 1 or 2, **characterized in that** the beam is made up of an assembly of parts (1, 8, 9) made of sheet steel.

5. A system according to claim 4, **characterized in that** said beam includes two parallel arms (1), a cylinder mount (9) secured to the cylinder (100), and a nozzle gate mount (8) secured to the manifold (200).

6. A system according to claim 5, **characterized in that** the arms (1) and the nozzle gate and cylinder mounts (8, 9) have complementary cutouts (17, 19, 83a, 84a) allowing them to interlock.

7. A system according to either claim 5 or 6, **characterized in that** the arms (1) and the nozzle gate mount (8) have cutouts (18, 81) allowing them to be centered on the nozzle base (300).

8. A system according to one of claims 4 to 7 **characterized in that** the lever includes two arms (2a, 2b) made of sheet steel.

9. A system according to one of claims 4 to 8, **characterized in that** the thickness of the sheet metal parts (1, 2, 8, 9) lies between 2 and 10 mm, preferably between 3 and 5 mm.

10. Sliding control device for the nozzle gate of an injection valve particularly for an injection system according to one of claims 1 to 9, **characterized in that** it includes:
- a flattened beam for holding the cylinder (100) in an offset position on the manifold (200), and
- a lever arranged so as to tilt about a trunnion (4) so as to transmit to the nozzle gate (7) the movements of the cylinder rod.

11. A manufacturing method for the system according to one of claims 4 to 9, **characterized in that** it includes the following steps:
(a) cutting out and, if necessary, bending of the parts (1, 8, 9) making up the beam,
(b) assembly of said parts and of the lever,
(c) securing of the assembly to the cylinder (100) and the manifold (200).

12. A method according to claim 11, **characterized in that** step (a) also includes cutting out the lever arms (2a, 2b).

13. A method according to either claim 11 or 12, **characterized in that** step (a) includes laser cutting.

14. A method according to one of claims 11 to 13, **characterized in that** step (b) is carried out by interlocking the parts.
